# EUROPEAN PATENT SPECIFICATION

(11) **EP 2 777 703 B1**
(45) Date of publication and mention of the grant of the patent: **14.09.2016**
(21) Application number: 13159548.0
(22) Date of filing: 15.03.2013
(51) Int. Cl.: A61K 31/437, A61K 35/28

(54) **Compositions and methods for enhancing the therapeutic potential of stem cells**
Zusammensetzungen und Verfahren zur Verbesserung des therapeutischen Potenzials von Stammzellen
Compositions et procédés permettant d'améliorer le potentiel thérapeutique de cellules souches

(43) Date of publication of application: 17.09.2014
(73) Proprietor: Lonza Cologne GmbH, 50829 Köln (DE)
(72) Inventor: van den Bos, Christian, 48268 Greven (DE); Reinisch, Barbara, 51375 Leverkusen (DE); Rosenbaum, Claudia, 40591 Düsseldorf (DE); Schenk, Judith, 50678 Köln (DE)
(74) Representative: Gassner, Birgitta

(56) References cited:
- WO-A2-2012/062819
- US-A1- 2010 216 181

## Description

### FIELD OF THE INVENTION

The invention relates to stem cell compositions and methods.

### BACKGROUND OF THE INVENTION

Stem cell therapy is a promising approach in bone marrow, skin, heart, and corneal transplantation, graft versus host disease, hepatic and renal failure, lung injury, rheumatoid arthritis, treatment of autoimmune diseases such as Crohn's disease, ulcerative colitis, multiple sclerosis, lupus and diabetes; prevention of allograft rejection, neurological disorders and cardiovascular medicine.

Mesenchymal stem cells (MSCs) and/or progenitor or precursor cells can be isolated from a variety of tissues, such as bone marrow, skeletal muscle, dental pulp, bone, umbilical cord and adipose tissue. MSCs are gaining increasing interest as a potential source for therapeutic approaches in regenerative medicine. Stem cells can self-renew and have the capacity to differentiate into specific cell types or can be therapeutically used for the production and secretion of soluble factors that will promote tissue regeneration. In particular, adult stem/progenitor cells from abundant autologous origin have the additional advantages over embryonic stem ("ES") cell types given their great availability, low tumorgenicity and allowing for the avoidance of controversial ethical issues associated with ES cells.

To facilitate an ability to efficiently repair or regenerate defective or diseased organs and tissues, donor stem/progenitor cells should possess desirable therapeutic properties, for example, minimal side effects, ability to integrate into host tissue, differentiation into desired cell lineages, paracrine effects, regulation of tissue remodeling and activation of endogenous repair/regeneration mechanisms.

MSCs have been employed in numerous preclinical studies in rodents, rabbits and baboon monkeys among others, for bone marrow, skin, heart, and corneal transplantation, graft versus host disease, hepatic and renal failure, lung injury, multiple sclerosis, rheumatoid arthritis, diabetes and lupus diseases. Preliminary results from some of these studies have led to human clinical trials that are currently being carried out. These trials involve treatment of autoimmune diseases such as Crohn's disease, ulcerative colitis, multiple sclerosis and type 1 diabetes mellitus; prevention of allograft rejection; and enhancement of the survival of bone marrow and kidney grafts; and treatment of resistant graft versus host disease.

A major component of morbidity and mortality attributable to cardiovascular disease occurs as a consequence of the partial or complete blockage of vessels carrying blood in the coronary and/or peripheral vasculature. When such vessels are partially occluded, lack of blood flow causes ischemia to the muscle tissues supplied by such vessel, consequently inhibiting muscle contraction and/or per function. Total occlusion of blood flow causes necrosis of the muscle tissue.

Peripheral artery disease (PAD) affects 8 million Americans. It is a result of narrowed arteries in the legs. The most common symptoms of PAD are cramping, pain or tiredness when walking. However, some PAD patients can lose the ability to walk and may suffer from amputations.

In some individuals, blood vessel occlusion is partially compensated by natural processes, in which new vessels are formed (a process often referred to as "angiogenesis") and small vessels are enlarged (termed "arteriogenesis") to replace the function of the impaired vessels. These new conduits may facilitate restoration of blood flow to the deprived tissue, thereby constituting "natural bypasses" around the occluded vessels.

However, some individuals with impaired angiogenesis are unable to generate sufficient collateral vessels to adequately compensate for the diminished blood flow caused by cardiovascular disease. There is a need for new therapeutic approaches to treat ischemic diseases.

Various studies have evaluated the therapeutic effect of MSCs in preclinical animal models and demonstrated great clinical potential. However, there remain major questions concerning optimal MSC dosage, route of administration, and MSC cell fate following infusion.

As such, there is a great need for optimized therapeutic MSC compositions and methods of using them, to ameliorate the symptoms of various diseases in general and in the treatment of vascular disease in particular.

Walker et al., Nat Commun. Author manuscript; available in 2010, generally disclose that treating human pluripotent stem (hPS) cells such as human embryonic stem (hES) and induced pluripotent stem (hiPS) cells with the inhibitor of non-muscle myosin II (NMII), blebbistatin, enhances the survival of hPS cells under clonal density and suspension conditions. U.S. Pub. No. 20100216181 discloses cultivation of pluripotent stem cells in a medium that is free of serum and feeder cells using blebbistatin as survival factor. Increased numbers of cells are generated by cultivating the cells in spinner flasks or bioreactors. Published PCT application WO2012062819 discloses a method for controlling binding of cells to a substrate using blebbistatin as a promoter for adhesion of cells to which the cells usually have no or limited affinity.

However, none of these references teach or suggest the unexpectedly superior results observed when treating patients with a composition containing stem cells and a non-muscle myson II antagonist.

### SUMMARY OF THE INVENTION

One aspect of the invention relates to a method of treating a vascular disease comprising administering to a patient in need thereof, an effective amount of a composition comprising a population of cells and blebbistatin, thus treating the vascular disease.

In one embodiment, the vascular disease is a result of diabetes. In another embodiment, the vascular disease is a result of atherosclerosis. In a further embodiment, the vascular disease is peripheral vascular disease (PVD). In still another embodiment, the vascular disease is peripheral artery disease (PAD). In still a further embodiment, the vascular disease is associated with an ankle brachial pressure index of about or less than 0.9. In yet another embodiment, the vascular disease is associated with an ankle brachial pressure index of about or less than 0.7. In still another embodiment, the vascular disease has resulted in critical limb ischemia. In another embodiment, the vascular disease has resulted in skin ulcerations or gangrene. In a further embodiment, the composition results in or enhances angiogenesis. In still another embodiment, the composition results in or enhances angiogenesis by at least about 20 percent compared to control.

In yet another embodiment, a portion of the population of cells comprises pluripotent cells. In still a further embodiment, the pluripotent cells are induced pluripotent stem cells. In another embodiment, a portion of the population of cells comprises multipotent cells. In still another embodiment, the multipotent cells are multipotent stromal cells or mesenchymal stem cells. In yet a further embodiment, the multipotent cells are derived from induced pluripotent stem cells. In still another embodiment, the composition comprises a pharmaceutically acceptable carrier.

In a further embodiment, the limb function in the patient improves by about or at least 2-3 grades compared to the same patient not receiving the composition. In another embodiment, the limb blood flow in the patient improves to about or at least 65-85% of normal or untreated limb blood flow. In yet a further embodiment, the ischemic damage in the patient improves by about or at least 2, 3 or 4 grades compared to the same patient not receiving the composition. In yet a further embodiment, the ischemic damage in the patient improves at a faster rate (or accelerated rate) compared to the same patient not receiving the composition.

In yet a further embodiment, the composition is administered intravenously. In still another embodiment, the composition is administered intramuscularly. In another embodiment, the composition is administered intramuscularly at or in proximity to a site of ischemic damage.

Another aspect of the invention relates to a kit comprising: a population of multipotent cells; blebbistatin a pharmaceutically acceptable carrier; and optionally, instructions for administering these to a patient diagnosed with disease which is amenable to treatment using stem cell thereapy. In one embodiment, the disease is a cardiovascular disease. In another embodiment, the disease is peripheral artery disease.

In one embodiment, the cells are MSCs. In another embodiment, the non-muscle myosin II antagonist is blebbistatin. In another embodiment, the cardiovascular disease is peripheral artery disease. In a further embodiment, the cells are MSCs, the non-muscle myosin II antagonist is blebbistatin and the cardiovascular disease is peripheral artery disease. In still another embodiment, the kit further comprises a cardiovascular drug. In a further embodiment, the kit further comprises a stent. In still another embodiment, the kit further comprises a drug-eluting stent. In one embodiment, the items comprising the kit are in separate containers. In another embodiment, the population of multipotent cells; the non-muscle myosin II antagonist; and the pharmaceutically acceptable carrier are in the same container, e.g., a syringe or automated administration device. The kit may be for a peripheral artery disease. The kit may have cells that are MSCs. The non-muscle myosin II antagonist is ideally blebbistatin and the disease is a peripheral artery disease. The kit may further comprises a cardiovascular drug, and/or a stent and/or a drug-eluting stent. The items may be in separate containers, in the same container or, in a syringe.

Another aspect of the invention relates to a method of reducing the rate of amputation in a peripheral artery disease patient treated with a composition comprising MSCs, comprising contacting the MSCs wherein, the non-muscle myosin II antagonist is blebbistatin. In another embodiment, the rate is reduced by at least about 20%, 30%, 40%, 50%, 60%, 70%, 80%, or 90% or more.

Another aspect of the invention relates to a stem cell composition for treating a disease amenable to treatment with stem cell therapy comprising a population of multipotent cells; and a non-muscle myosin II antagonist, wherein the non-muscle myosin II antagonist is blebbistatin. In one embodiment of this aspect of the invention, the cells are MSCs.

Another aspect of the invention relates to a method of making a stem cell composition for treating a disease amenable to treatment with stem cell therapy comprising a population of multipotent cells; and the non-muscle myosin II antagonist blebbistatin; wherein the population of multipotent cells and blebbistatin are incubated together for period of time prior to administration to a patient.

A further aspect of the invention is directed to a method of accelerating the healing of a disease amenable to treatment with stem cell therapy comprising a treating a patient with a composition comprising a population of multipotent cells; and the non-muscle myosin II antagonist blebbistatin; wherein the composition results in healing of the disease faster than the same composition lacking the non-muscle myosin II antagonist blebbistatin.

In certain embodiments of these aspects, the cells are MSCs. In other embodiments, the NM II antagonist is blebbistatin. In yet further embodiments, the disease are a cardiovascular disease.

### BRIEF DESCRIPTION OF THE FIGURES

A more complete understanding of the present invention may be obtained by reference to the accompanying drawings, when considered in conjunction with the subsequent detailed description. The embodiments illustrated in the drawings are intended only to exemplify the invention and should not be construed as limiting the invention to the illustrated embodiments.

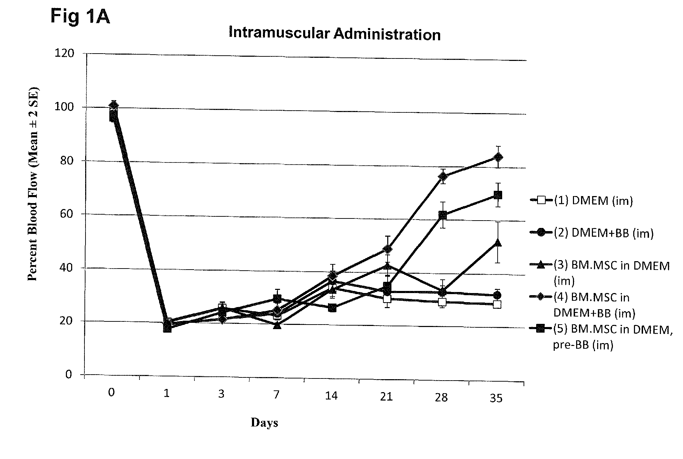

Figures 1A-B show Blood Flow (%) over Time: The median in the group was computed across all valid animals. Blebbistatin (BB) accelerates and enhances blood flow regeneration by mesenchymal stem cell implantation. Stem cells administered in the presence of blebbistatin (1A, Group 4, diamond) showed faster and enhanced regeneration in blood flow in animals compared to stem cells that have been pre-treated with blebbistatin but were implanted in the absence of blebbistatin (1A, Group 5, solid black square). Media control containing blebbistatin (1A, Group 2, circle) showed marginally recovery effects as well as MSCs, which picked up on recovery at later time points (1A, Group 3, triangle). Intramuscular application of MSCs showed consistently superior effects over intravenous application. The data show that blebbistatin (BB) alone had no effect on the regeneration of blood flow but blebbistatin enhances and/or supports the regenerative potential of MSCs when applied together with stem cells.

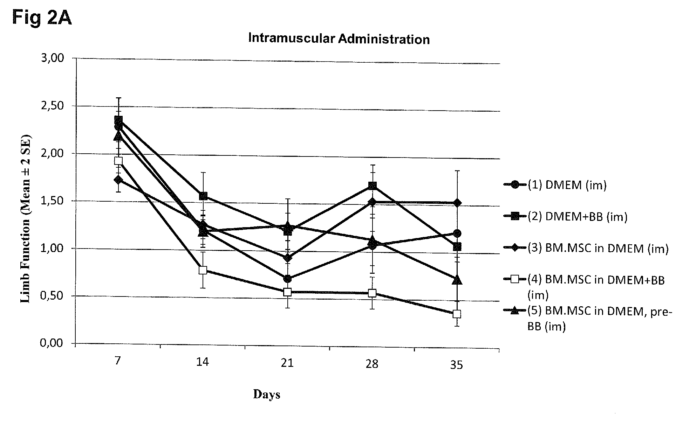

Figure 2A-B show Limb Function (%) over Time: The median in the group was computed across all valid animals. The data show that blebbistatin (BB) presence with MSCs supports fast improvement in limb function. Despite the blood perfusion improvement in other treated groups, limb functional deterioration and limb necrosis increase was observed from day 28 after the treatment. Group 3 exhibited larger occurrence of amputations and larger distribution between animals. The data show that intravenous treatment (2B) was somewhat less effective and caused more complications compared to the local intramuscular treatment regime (Figure 2A). Mice that have received MSCs implanted in the presence of blebbistatin (2A, Group 4, open square) showed constant and fast improvement of limb function consistently over time after implantation. The same tendency was detected in mice with blebbistatin-pretreated MSCs to a lower amount (2A, Group 5, triangle). In contrast, the application of MSCs alone showed slight improvement until day 20 and limb function worsened at later time points. Media controls exhibited marginal effects only. Note, that a negative slope means improvement of the limb function. Again, intramuscular application of MSCs (2A) showed consistently superior effects over intravenous application (2B). Application of MSCs in the presence of blebbistatin outperforms the regenerative potential of MSCs. This application showed the fastest improvement in regenerative potential.

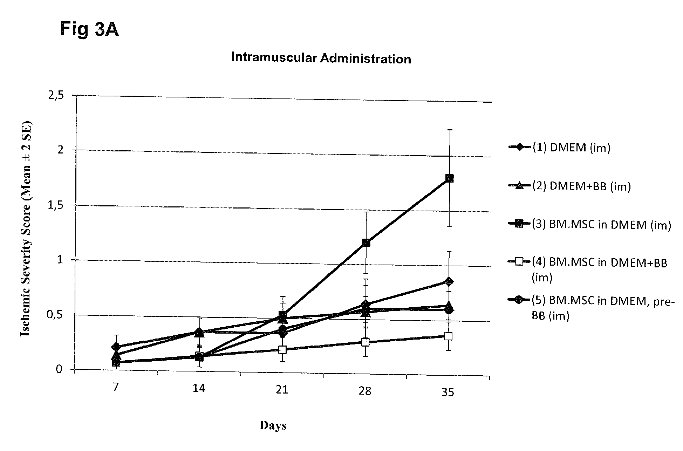

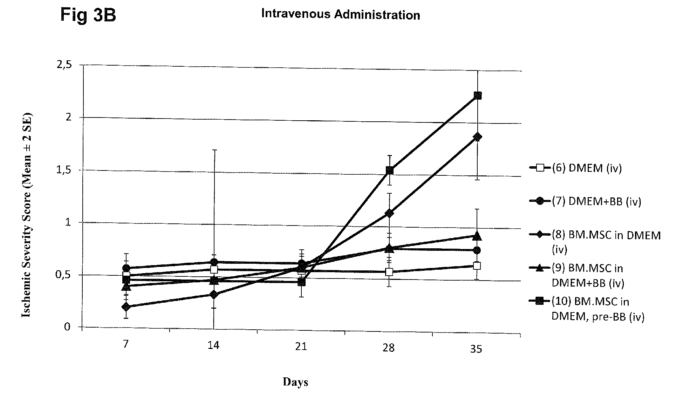

Figure 3A-B show Ischemic Severity Score (%): The median in the group was computed across all valid animals. MSCs applied in conjunction with blebbistatin impede severity in Ischemic Score. Analysis of the ischemic severity score over time show consistently better results for Group 4 and 5 when applied intramuscularly, although media controls ranged within the scatter. Note that MSCs (Group 3) showed a high variance in Score for both intramuscular and intravenous applications as Group 5 for the intravenous application only. Again, intramuscular application of MSCs is shown in 3A; intravenous application is shown in 3B.

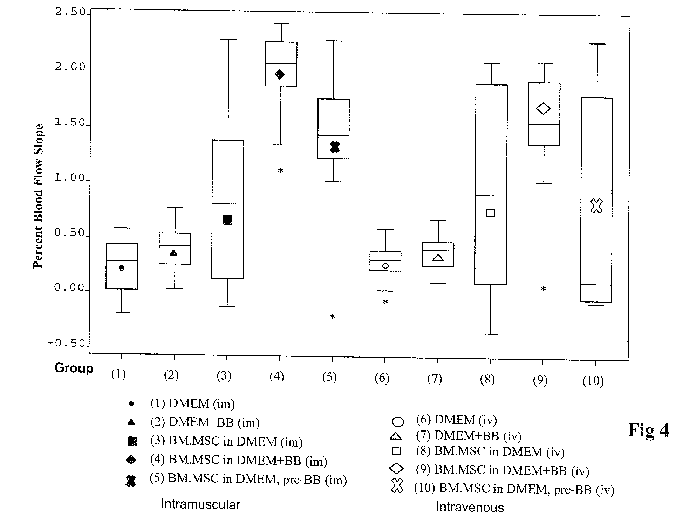

Figure 4 shows the distribution of box plot slopes for Blood Flow (%), computed as the linear trend from surgery to Day 35 for Blood Flow (%). The single number for each animal represents its change on an endpoint over time. These plots provide a convenient method for presenting many groups on a single graph. Exemplary, the evaluation of blood flow (%) is depicted as slopes boxplots by group in order to point out the low variation (indicated by the small box sizes) within controls and the best performing group in the study BMMSC in DMEM + BB for intramuscular as well as intravenous application.

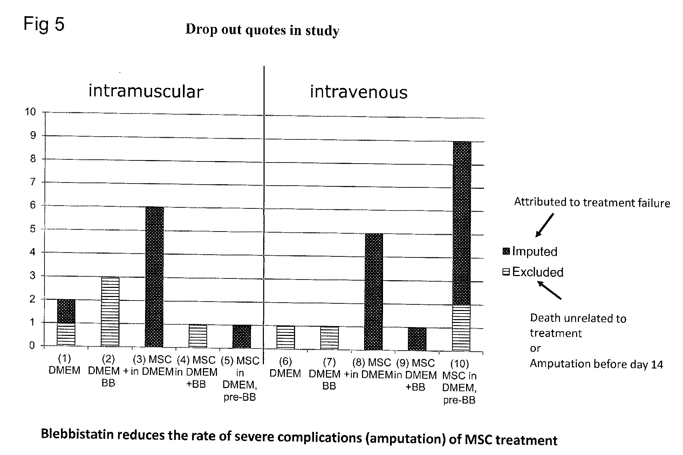

Figure 5 shows blebbistatin impedes severe complications during the healing process. A major problem in pre-clinical studies is often the high drop-out rate of animals due to treatment related failure (amputations after day 14 post surgery) or unrelated to treatment (e.g. death during anesthesia, or amputations before day 14 post surgery). Only a few animals had to be excluded from statistical analysis due to the latter reason. However, those animals that showed severe side effects (amputation) were ranked and their data were imputed in the statistical analyses. Data clearly demonstrate that blebbistatin (Groups 4 and 8) drastically improves regenerative potential of MSCs and stand out by strikingly low rates of severe complications.

### DETAILED DESCRIPTION OF THE INVENTION

Described herein are MSC compositions and methods that allow for faster healing and enhanced regeneration of diseased or damaged tissues while strongly reducing complications during healing. In particular, it is disclosed herein that inventive MSCs compositions have great regenerative potential in a validated animal model. In particular, it has been found that blebbistatin dramatically and unexpectedly accelerate and enhances the therapeutic potential of MSCs and reduces severe complications in healing.

The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes the inherent variation of error for the device, the method being employed to determine the value, or the variation that exists among the study subjects. Typically the term is meant to encompass approximately or less than 1%, 2%, 3%, 4%, 5%, 6%, 7%, 8%, 9%, 10%, 11%, 12%, 13%, 14%, 15%, 16%, 17%, 18%, 19% or 20% variability depending on the situation.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. It is contemplated that any embodiment discussed in this specification can be implemented with respect to any method or composition of the invention, and vice versa. Furthermore, compositions of the invention can be used to achieve methods of the invention.

"Treating," "treat" or "treatment" is referred to herein as administration of a substance to a subject with the purpose to cure, alleviate, relieve, remedy, prevent, or ameliorate a disorder, symptoms of the disorder, a disease state secondary to the disorder, or predisposition toward the disorder. An "effective amount" is an amount of the substance that is capable of producing a medically desirable result as delineated herein in a treated subject. The medically desirable result may be objective (i.e., measurable by some test or marker) or subjective (i.e., subject gives an indication of or feels an effect).

"Disease amenable to treatment with stem cell therapy" as referred to herein means any procedures, conditions, disorders, ailments and/or illnesses which can be treated by the administration of stem cells. Such diseases include but are not limited to bone marrow, skin, heart, and corneal transplantation, graft versus host disease, hepatic and renal failure, lung injury, rheumatoid arthritis, treatment of autoimmune diseases such as Crohn's disease, ulcerative colitis, multiple sclerosis, lupus and diabetes; prevention of allograft rejection, neurological disorders and cardiovascular medicine; as well as Acute lymphoblastic leukemia (ALL), Acute myeloid leukemia (AML), Burkitt's lymphoma, Chronic myeloid leukemia (CML), Juvenile myelomonocytic leukemia (JMML), Non-Hodgkin's lymphoma Hodgkin's lymphoma, Lymphomatoid granulomatosis, Myelodysplastic syndrome (MDS), Chronic myelomonocytic leukemia (CMML), Bone Marrow Failure Syndromes, Amegakaryocytic thrombocytopenia, Autoimmune neutropenia (severe), Congenital dyserythropoietic anemia, Cyclic neutropenia, Diamond-Blackfan anemia, Evan's syndrome, Fanconi anemia, Glanzmann's disease, Juvenile dermatomyositis, Kostmann's syndrome, Red cell aplasia, Schwachman syndrome, Severe aplastic anemia, Congenital sideroblastic anemia, Thrombocytopenia with absent radius (TAR syndrome), Dyskeratosis congenital, Blood Disorders, Sickle-cell anemia (hemoglobin SS), HbSC disease, Sickle βo Thalassemia, α-thalassemia major (hydrops fetalis), β-thalassemia major (Cooley's anemia), β-thalassemia intermedia, E-βo thalassemia, E-β+ thalassemia, Metabolic Disorders, Adrenoleukodystrophy Gaucher's disease (infantile), Metachromatic leukodystrophy, Krabbe disease (globoid cell leukodystrophy), Gunther disease, Hermansky-Pudlak syndrome, Hurler syndrome, Hurler-Scheie syndrome, Hunter syndrome, Sanfilippo syndrome, Maroteaux-Lamy syndrome, Mucolipidosis Type II, III, Alpha mannosidosis, Niemann Pick Syndrome, type A and B, Sandhoff Syndrome, Tay-Sachs Disease, Batten disease (inherited neuronal ceroid lipofuscinosis), Lesch-Nyhan disease, Immunodeficiencies, Ataxia telangiectasia, Chronic granulomatous disease, DiGeorge syndrome, IKK gamma deficiency, Immune dysregulation polyendocrineopathy, X-linked Mucolipidosis, Type II, Myelokathexis X-linked immunodeficiency, Severe combined immunodeficiency, Adenosine deaminase deficiency, Wiskott-Aldrich syndrome, X-linked agammaglobulinemia, X-linked lymphoproliferative disease, Omenn's syndrome, Reticular dysplasia, Thymic dysplasia, Leukocyte adhesion deficiency, Other Osteopetrosis, Langerhans cell histiocytosis, Hemophagocytic lymphohistiocytosis, Acute & Chronic Kidney Disease, Alzheimer's disease, Anti-Aging, Arthritis, Asthma, Cardiac Stem Cell Therapy, Cerebral Infarction (Stroke), Cerebral Palsy (Stroke), Chronic Obstructive Pulmonary Disease (COPD), Congestive Heart Failure, Diabetes Mellitus (Type I & II), Fibromyalgia, Immune Deficiencies, Ischemic Heart Disease, Lupus, Multiple Sclerosis, Myocardial Infarction, Osteoarthritis, Osteoporosis, Parkinson's Disease, Peripheral Arterial Disease, Rheumatoid Arthritis, Stem Cell Therapy in Plastic Surgery, Traumatic Brain Injury and Neurological Diseases.

"Vascular or cardiovascular disease" as referred to herein is characterized by clinical events including clinical symptoms and clinical signs. Clinical symptoms are those experiences reported by a patient that indicate to the clinician the presence of pathology. Clinical signs are those objective findings on physical or laboratory examinations that indicate to the clinician the presence of pathology. "Cardiovascular disease" includes both "coronary artery disease" and "peripheral vascular disease," both terms being defined below. Clinical symptoms in cardiovascular disease include chest pain, shortness of breath, weakness, fainting spells, alterations in consciousness, extremity pain, paroxysmal nocturnal dyspnea, transient ischemic attacks and other such phenomena experienced by the patient. Clinical signs in cardiovascular disease include such findings as EKG abnormalities, altered peripheral pulses, arterial bruits, abnormal heart sounds, rales and wheezes, jugular venous distention, neurological alterations and other such findings discerned by the clinician. Clinical symptoms and clinical signs can combine in a cardiovascular disease such as a myocardial infarction (MI) or a stroke (also termed a "cerebrovascular accident" or "CVA"), where the patient will report certain phenomena (symptoms) and the clinician will perceive other phenomena (signs) all indicative of an underlying pathology. "Cardiovascular disease" includes those diseases related to the cardiovascular disorders of fragile plaque disorder, occlusive disorder and stenosis. For example, a cardiovascular disease resulting from a fragile plaque disorder, as that term is defined below, can be termed a "fragile plaque disease." Clinical events associated with fragile plaque disease include those signs and symptoms where the rupture of a fragile plaque with subsequent acute thrombosis or with distal embolization are hallmarks. Examples of fragile plaque disease include certain strokes and myocardial infarctions. As another example, a cardiovascular disease resulting from an occlusive disorder can be termed an "occlusive disease." Clinical events associated with occlusive disease include those signs and symptoms where the progressive occlusion of an artery affects the amount of circulation that reaches a target tissue. Progressive arterial occlusion may result in progressive ischemia that may ultimately progress to tissue death if the amount of circulation is insufficient to maintain the tissues. Signs and symptoms of occlusive disease include claudication, pain while resting, angina, and gangrene, as well as physical and laboratory findings indicative of vessel stenosis and decreased distal perfusion. As yet another example, a cardiovascular disease resulting from restenosis can be termed an in-stent stenosis disease. In-stent stenosis disease includes the signs and symptoms resulting from the progressive blockage of an arterial stent that has been positioned as part of a procedure like a percutaneous transluminal angioplasty, where the presence of the stent is intended to help hold the vessel in its newly expanded configuration. The clinical events that accompany in-stent stenosis disease are those attributable to the restenosis of the reconstructed artery.

A "coronary artery disease" ("CAD") refers to a vascular disorder relating to the blockage of arteries serving the heart. Blockage can occur suddenly, by mechanisms such as plaque rupture or embolization. Blockage can occur progressively, with narrowing of the artery via myointimal hyperplasia and plaque formation. Those clinical signs and symptoms resulting from the blockage of arteries serving the heart are manifestations of coronary artery disease. Manifestations of coronary artery disease include angina, ischemia, myocardial infarction, cardiomyopathy, congestive heart failure, arrhythmias and aneurysm formation. It is understood that fragile plaque disease in the coronary circulation is associated with arterial thrombosis or distal embolization that manifests itself as a myocardial infarction. It is understood that occlusive disease in the coronary circulation is associated with arterial stenosis accompanied by anginal symptoms, a condition commonly treated with pharmacological interventions and with angioplasty.

"Peripheral Vascular Disease" or ("PVD") is a cardiovascular disease resulting from the blockage of the peripheral (i.e., non-coronary) arteries. Blockage can occur suddenly, by mechanisms such as plaque rupture or embolization, as occurs in fragile plaque disease. Blockage can occur progressively, with narrowing of the artery via myointimal hyperplasia and plaque formation, as in occlusive disease. Blockage can be complete or partial. Those clinical signs and symptoms resulting from the blockage of peripheral arteries are manifestations of peripheral vascular disease. Manifestations of peripheral vascular diseases include, inter alia, claudication, ischemia, intestinal angina, vascular-based renal insufficiency, transient ischemic attacks, aneurysm formation, peripheral embolization and stroke. Ischemic cerebrovascular disease is a type of peripheral vascular disease.

Upon suspicion of PVD, the ankle brachial pressure index (ABPI/ABI) may be determined. When the blood pressure readings in the ankles are lower than that in the arms, blockages in the arteries which provide blood from the heart to the ankle may be suspected. An ABI ratio of about or less than 0.9 consistent with PVD; values of ABI about or below 0.8 indicate moderate disease and about or below 0.5 imply severe ischemic disease. An ABI ratio of about or less than 0.5 or 0.4 may be used as a threshold for diagnosis.

It is possible for conditions which stiffen the vessel walls (such as calcifications that occur in the setting of chronic diabetes) to produce false negatives usually, but not always, indicated by abnormally high ABIs (about or > 1.3). Such results and suspicions merit further investigation and higher level studies. If ABIs are abnormal the next step is generally a lower limb doppler ultrasound examination to look at site and extent of atherosclerosis. Other imaging may be performed by angiography, where a catheter is inserted into the common femoral artery and selectively guided to the artery in question. While injecting a radiodense contrast agent an X-ray may be taken. Any flow limiting stenoses found in the x-ray can be identified and treated by atherectomy, angioplasty or stenting. Multislice computerized tomography (CT) scanners may provide direct imaging of the arterial system as an alternative to angiography. CT may facilitate evaluation of the aorta and lower limb arteries without the need for an angiogram's arterial injection of contrast agent.

Peripheral artery disease (PAD) is a form of peripheral vascular diseases (PVD) in which there are partial or total blockage of blood supply to a limb, usually the leg, leading to impaired blood flow and hypoxia in the tissue. When PAD advances it reaches the stage of critical limb ischemia (CLI) with skin ulcerations, gangrene and potentially unavoidable amputations. Hind limb ischemia animal models have been used to evaluate various therapeutic approaches addressing stem cell transplantation.

The current PAD standard of care includes smoking cessation, reduction in cholesterol, antiplatelet agents, treatment of diabetes, treatment of high blood pressure, ACE-inhibitors, exercise, and cilostazol. Additional treatment options can involve stents, e.g., drug-eluting stents. Such as paclitaxel-eluting stents. The methods and compositions disclosed herein are all envisaged to be provided with such treatment options. See for example, Bums et al., BMJ. 2003 March 15; 326(7389): 584-588.

Peripheral artery disease is commonly divided in the Fontaine stages, introduced by René Fontaine in 1954 for ischemia: 1) Mild pain when walking (claudication), incomplete blood vessel obstruction; 2) Severe pain when walking relatively short distances (intermittent claudication), pain triggered by walking "after a distance of >150 m in stage II-a and after <150 m in stage II-b"; 3) Pain while resting (rest pain), mostly in the feet, increasing when the limb is raised; 4) Biological tissue loss (gangrene) and difficulty walking. The compositions and methods disclosed herein are envisaged to result in the improvement of peripheral artery disease by improving the Fontaine Stage of a patient by about or at least 1, 2, 3 or 4 Stages over a period of at least or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 hours, days, weeks or months from the initiation of treatment or compared to control.

Alternatively, classification of peripheral artery disease by Rutherford consists of six stages: 1) Mild claudication; 2) Moderate claudication; 3) Severe claudication; 4) Ischemic pain at rest; 5) Minor tissue loss; and 6) Major tissue loss. The compositions and methods disclosed herein are envisaged to result in the improvement of peripheral artery disease by improving the Rutherford Stage of a patient by about or at least 1, 2, 3, 4, 5 or 6 Stages over a period of at least or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 hours, days, weeks or months from the initiation of treatment or compared to control.

In one embodiment of the invention the composition claimed herein results in or enhances angiogenesis.

Ideally, the composition results in or enhances angiogenesis by at least 20 percent compared to control.

In one embodiment of the invention the composition claimed herein comprises a pharmaceutically acceptable carrier.

"Assessment of limb function" is referred to herein as a relative measurement of the effectiveness of the methods and compositions disclosed herein. Alternatively, limb function grades are: "0" for flexing the toes to resist gentle traction of the tail, "1" for plantar flexion, "2" for dragging but no plantar flexion, and "3" for dragging of foot. The compositions and methods disclosed herein are envisaged to result in the improvement in limb function of a patient by about or at least 1, 2, 3 or 4 grades over a period of at least or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 hours, days, weeks or months from the initiation of treatment or compared to control.

"Assessment of ischemic damage" can be used to evaluate various therapeutic approaches addressing stem cells transplantation. In an embodiment, ischemic damage can be expressed as a morphological grade. The morphological grades are: "0" for substantial absence of necrosis, "1" for necrosis substantially limiting to toes (toes loss), "2" for necrosis extending to a dorsum pedis (foot loss), "3" for necrosis extending to a crus (knee loss), and "4" for necrosis extending to a thigh (total hind-limb loss). The compositions and methods disclosed herein are envisaged to result in the reduction of ischemic damage in a patient by about or at least 1, 2, 3 or 4 grades over a period of at least or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 hours, days, weeks or months from the initiation of treatment or compared to control.

"Assessment in limb blood flow" is referred to herein as an additional relative measurement of the effectiveness of the methods and compositions disclosed herein. Preferably, blood flow in legs from both sides is measured, e.g., with a non-contact Laser Doppler, before and after treatment. The compositions and methods disclosed herein are envisaged to result in the improvement in limb blood flow of a patient to about or at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99.5 or 100 percent of normal blood perfusion levels over a period of at least or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 hours, days, weeks or months from the initiation of treatment or compared to control.

"Reducing the rate of amputation in a peripheral artery disease patient" refer to the rate of PAD patients requiring amputation of limbs, digits and/or tissues due to e.g., infection or necrosis. There are standard rates of amputation associated with treatment with MSCs which can be readily determined. Preferably, the treatment of patients with a composition which includes a combination of MSCs and an NM II antagonists reduces the rates of such amputation by about or at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99.5, 100, 125, 150, 175, 200, 250, 300, 350, 400, 500 or more percent relative to a standard (e.g., standard of care) or control (e.g., MSC treatment or NM II antagonist treatment alone but not in combination).

"Cardiovascular Disease Drugs" as defined herein refers to medicaments useful for the management of diabetes, hypertension (e.g., ACE-inhibitors), cholesterol (e.g., statins), and antiplatelet drugs (e.g., aspirin, clopidogrel), anti-coagulants (e.g., warfarin), diuretics and beta-blockers. Additionally, cilostazol or pentoxifylline are cardiovascular diabetes drugs. The term "angiogenesis" is defined as a physiological process involving the growth of new blood vessels from pre-existing vessels. Vasculogenesis is the term used for spontaneous blood-vessel formation, and intussusception is the term for new blood vessel formation by splitting off existing ones. Angiogenesis is a normal process in growth and development, as well as in wound healing. It is also a fundamental step in the transition of tumors from a dormant state to a malignant state. Angiogenesis is said to be taking place when there is about or at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99.5, 100, 125, 150, 175, 200, 250, 300, 350, 400, 500 or more percent or more increase in the growth of new blood vessels in a given area when compared with the same tissue prior to angiogenesis or to a standard (e.g., standard of care) or control (e.g., MSC treatment or NM II antagonist treatment alone but not in combination).

"Accelerating healing" refers to the increased speed at which a disease amenable to stem cell therapy shows signs of improvement (with respect to a well-excepted qualitative or quantitative morphological or functional measurement associated with the disease in question) in response to therapy with the compositions disclosed herein. Preferably, the speed of healing increases by about or at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 99.5, 100, 125, 150, 175, 200, 250, 300, 350, 400, 500 or more percent a standard (e.g., standard of care) or control (e.g., MSC treatment or NM II antagonist treatment alone but not in combination).

"Patient" as used herein refers to a mammalian subject diagnosed with or suspected of having or developing cardiovascular disease. Exemplary patients may be humans, apes, dogs, pigs, cattle, cats, horses, goats, sheep, rodents and other mammalians that can benefit from stem cell therapies.

"Administering" is referred to herein as providing the compositions of the invention to a patient. By way of example and not limitation, composition administration, e.g., injection, may be performed by intravenous (i.v.) injection, sub-cutaneous (s.c.) injection, intradermal (i.d.) injection, intraperitoneal (i.p.) injection, or intramuscular (i.m.) injection. One or more such routes may be employed. Parenteral administration can be, for example, by bolus injection or by gradual perfusion over time. Alternatively, or concurrently, administration may be by the oral route. Additionally, administration may also be by surgical deposition of a bolus or pellet of cells, or positioning of a medical device, e.g., a stent, loaded with cells. Preferably, the compositions of the invention are administered at the site of ischemic cardiovascular disease, e.g. at the site or near (e.g., about or at least 1, 2, 3, 4, 5, 6, 7, 8, 9, 10, 11, 12, 13, 14, 15, 16, 17, 18, 19, 20, 25, 30, 40, 50 millimeters from) the a of the ischemic cardiovascular disease lesion (e.g., vascular stenosis/blockage, necrotic tissue or site of gangrenous infection). Administration may be intravenous, intramuscular, at or near a site of a disease-associated lesion and/or intramuscular at or in proximity to the site of ischemic damage.

"A patient in need thereof' is referred to herein as a patient diagnosed with or suspected of having cardiovascular disease. In one embodiment, the patient has or is likely to develop peripheral artery disease.

"Totipotency" is referred to herein as the ability of a single cell to divide and/produce all the differentiated cells in an organism, including extra-embryonic tissues. Totipotent cells include spores and zygotes. In some organisms, cells can dedifferentiate and regain totipotency.

"Pluripotency" is referred to herein as the potential to differentiate into any of the three germ layers: endoderm (interior stomach lining, gastrointestinal tract, the lungs), mesoderm (muscle, bone, blood, urogenital), or ectoderm (epidermal tissues and nervous system).

"Pluripotent stem cells" include natural pluripotent stem cells and induced pluripotent stem cells. They can give rise to any fetal or adult cell type. However, alone they generally cannot develop into a fetal or adult organism because they lack the potential to contribute to extra-embryonic tissue, such as the placenta.

"Induced pluripotent stem cells" or ("iPS cells") are similar to natural pluripotent stem cells, such as embryonic stem (ES) cells, in many aspects, such as the expression of certain stem cell genes and/orteins, chromatin methylation patterns, doubling time, embryoid body formation, teratoma formation, viable chimera formation, and potency and differentiability. Induced pluripotent cells may be derived from for example, adult stomach, liver, skin cells and blood cells. iPS cells may be derived by transfection of certain stem cell-associated genes into non-pluripotent cells, such as adult fibroblasts. In certain embodiments, transfection may be achieved through viral vectors, such as retroviruses, for example. Transfected genes can include, but are not limited to, master transcriptional regulators Oct-3/4 (Pou5f1) and Sox2 Oct-4, Nanog and Lin28 transgenes. Sub-populations of transfected cells may begin to become morphologically and biochemically similar to pluripotent stem cells, and can be isolated through morphological selection, doubling time, or through a reporter gene and antibiotic selection.

"Multipotency" is referred to herein as multipotent progenitor cells which have the potential to give rise to multiple cell types, but a number of lineages more limited than a pluripotent stem cell. For example, a multipotent stem cell is a hematopoietic cell that can develop into several types of blood cells, but cannot develop into brain cells or other types of cells.

"Mesenchymal stem cells or Multipotent Stromal Cell" (both referred to as "MSCs") are referred to herein as being multipotent stromal cells that can differentiate into a variety of cell types, including but not limited to: osteoblasts (bone cells), chondrocytes (cartilage cells), and adipocytes (fat cells).

In one embodiment, mesenchymal stem cells are obtained from bone marrow. In another one embodiment, mesenchymal stem cells are obtained from developing tooth bud of the mandibular third molar. In another embodiment, MSCs are obtained from amniotic fluid. In another embodiment, MSCs may be obtained from the umbilical cord tissue, e.g., from Wharton's jelly and the umbilical cord blood. In a further embodiment, MSCs are isolated from adipose tissue. Alternatively, MSCs are isolated from Wharton's jelly. In another embodiment, MSCs are derived from iPS cells as, for example, described in Guiliani et al., "Human mesenchymal stem cells derived from induced pluripotent stem cells downregulate NK cell cytolytic machinery," Blood, July 29, 2011.

MSCs preferably have a small cell body with a few cell processes that are long and thin. The cell body may contain a large, round nucleus with a prominent nucleolus, which is surrounded by finely dispersed chromatin particles, giving the nucleus a clear appearance. The remainder of the cell body contains a small amount of golgi apparatus, rough endoplasmic reticulum, mitochondria, and polyribosomes. MSCs which are long and thin, are widely dispersed and the adjacent extracellular matrix is populated by a few reticular fibrils but is devoid of the other types of collagen fibrils.

Cell attachment is not a homogeneous process but rather occurs through particular zones, so-called focal adhesion zones. Once attached, cells exert tension by means of internal motor proteins. Thus, the cytoskeleton may become organized and functions in a variety of processes including transport, scaffolding and cell survival. Upon de-attaching cells, these may quickly constrict as a function of the internal tension build up by motor proteins and the previously organized internal structures (cytoskeletal and other) can be disturbed. If not allowed to re-attach to surfaces, such cells may initiate cell death programs which have been described as attachment-dependent apopotosis/anoikis.

"Myosins" include a family of ATP-dependent motor proteins and are generally known for their role in muscle contraction and their involvement in a wide range of other eukaryotic motility processes. They are generally responsible for actin-based motility. Generally, myosin II (also known as conventional myosin) is the myosin type responsible for producing muscle contraction in muscle cells.

The major cytoskeletal motor protein responsible for generating cell tension is non-muscle myosin II (referred to as myosin II). "Non-Muscle Myosin II" or "NM II" is an actin-binding protein that has actin cross-linking and contractile properties and is regulated in part through the phosphorylation of its light and heavy chains. Like muscle myosin II, NM II molecules are comprised of three pairs of peptides: two heavy chains of 230 kDa, two 20 kDa regulatory light chains (RLCs) that regulate NM II activity and two 17 kDa essential light chains (ELCs) that stabilize the heavy chain structure. Although these myosins are referred to herein as "non-muscle" myosin IIs to distinguish them from their muscle counterparts, they are also present in muscle cells, where they have distinct functions during skeletal muscle development and differentiation, as well as in the maintenance of tension in smooth muscle.

Generally, three mammalian NM II isoforms have both overlapping and unique properties. The two globular head domains of NM II contain a binding site for both ATP and actin and they are followed by neck regions, each of which binds the two functionally different light chains. The neck domain acts as a lever arm to amplify head rotation while the chemical energy of ATP is converted into the mechanical movement of the myosin head. This neck domain is followed by a long α-helical coiled coil, which forms an extended rod-shaped domain that effects dimerization between the heavy chains and terminates in a relatively short non-helical tail. The rod domains of NM II self-associate to form bipolar filaments (antiparallel arrays of myosin molecules), which are smaller than those found in cardiac and skeletal muscle.

NM II acts to integrate processes that drive cell migration and adhesion. It is also an important end point on which many signaling pathways converge, largely through Rho GTPases. NM II itself is tightly regulated at different levels, including at the level of folding, myosin filament assembly and disassembly, actin binding and ATPase and motor activity. The regulation of the actin cytoskeleton by NM II controls multiple interrelated processes, such as migration, cell-cell and cell-matrix adhesion, cell differentiation, tissue morphogenesis and development. The spatiotemporal regulation of NM II by subcellular localization and activation of its regulating kinases in different cells and tissues has important ramifications in controlling the NM II function.

The regulation of Mg²⁺-ATP hydrolysis and filament formation of NM II involves the reversible phosphorylation of specific amino acids present in the pair of 20 kDa RLCs and the heavy chains. The function of the ELC pair is to stabilize the NMHC and there is no evidence that they undergo reversible phosphorylation. See also Vicente-Manzanares et al., Nat Rev Mol Cell Biol. 2009 November; 10(11): 778-790.

"Non-Muscle Mysosin II antagonist" refers to an agent that directly or indirectly inhibits, blocks or reverses the activity of Myosin II as it occurs in nature or in the diseased or damaged tissues of patients suffering from cardiovascular disease. Such an agent can be a small molecule (e.g., blebbistatin); nucleic acid including rRNA, mRNA, tRNA, miRNA, siRNA (see e.g., Ma et al., Mol. Biol. Cell November 15, 2010 vol. 21 no. 22 3952-3962 or Kim et al, J Biol Chem. 2012 Aug 10;287(33):27345-58), anti-sense RNA, DNA, RNA/DNA hybrid, ribozyme, PNA, aptamer, (all or any of which may include synthetic or non-natural nucleotide bases); peptides; or proteins e.g., antibodies, antibody fragments, antibody-like molecules or single chain antibodies, as well as phosphatases and kinases. For example, NM II antagonist can be an siRNA targeted to knock down the expression of the NM II encoding mRNA or the mRNA of a gene associated with the up-regulation of NM II; or a vector encoding such an siRNA.

II antagonists include Angiotensin II Type 1 Receptor Antagonists, (e.g., the non-peptide AT1 receptor antagonist FK-739 see Fuji et al., Hypertension, 1999;33:975-980) as well as Angiotensin II receptor antagonists, also known as angiotensin receptor blockers (ARBs), AT1-receptor antagonists or sartans, are a group of pharmaceuticals which modulate the renin-angiotensin-aldosterone system. Their main uses are in the treatment of hypertension (high blood pressure), diabetic nephropathy (kidney damage due to diabetes) and congestive heart failure. Losartan, irbesartan, olmesartan, candesartan and valsartan include the tetrazole group (a ring with four nitrogen and one carbon). Losartan, irbesartan, olmesartan, candesartan, and telmisartan include one or two imidazole groups.

Non-Muscle Mysosin II antagonists also include ACE inhibitors. An ACE inhibitor (or angiotensin-converting-enzyme inhibitor) is a medication pharmaceutical drug used primarily for the treatment of high blood pressure (hypertension) and weak heart muscle (congestive heart failure). Exemplary ACE inhibitors include captopril, zofenopril, enalapril, ramipril, quinapril, perindopril, lisinopril, benazepril, imidapril, zofenopril, trandolapril, fosinopril, casokinins and lactokinins, as well as the lactotripeptides Val-Pro-Pro and Ile-Pro-Pro produced by the probiotic Lactobacillus helveticus or derived from casein have been shown to have ACE-inhibiting and antihypertensive functions.

Non-Muscle Mysosin II antagonists also include 2,3-butanedione-2-monoxime (BDM) and inhibitors of myosin II adenosine triphosphatase (ATPase) activity.

One NM II antagonist is blebbistatin (a 1-phenyl-1-2-pyrrolidinone derivative) which is a specific pharmacologic inhibitor of skeletal muscle and nonmuscle myosin II adenosine triphosphatase (ATPase) activity. Blebbistatin is highly active small molecule that inhibits cell blebbing. This compound is cell-permeant, benign and, importantly, readily reversible. It has been described as a survival factor in stem cell cultures. It has also been described as a promoter for adhesion of cells to a substrate to which the cells usually have no or only low affinity (WO2012062819), e.g. as for coating of implanted medical devices such as pace makers, hip or bone implants or stents.
Blebbistatin is shown below:

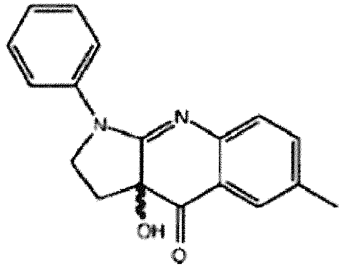

In some embodiments, the term "blebbistatin" includes a racemic mixture. In some embodiments, the term blebbistatin refers to R-blebbistatin. In some embodiments, the term blebbistatin refers to S-blebbistatin.

This disclosure encompasses blebbistatin variants, analogs and derivatives. Additional blebbistatin variants, analogs and derivatives and Non-Muscle Mysosin II antagonists suitable and envisaged for use herein are disclosed in U.S. Pub. No. 20080021035. See for example also: Straight et al., Science 299 (5613): 1743-47; Kovács et al., (Aug 2004), J Biol Chem. 279 (34): 35557-63; Limouze et al., (2004) J Muscle Res Cell Motil. 25 (4-5): 337-41.

In certain embodiments, the therapeutic compositions disclosed herein contain or are associated with, at least one Non-Muscle Mysosin II antagonist whereby each at least one Non-Muscle Mysosin II antagonist is present in single dose at a concentration of about, at least or more than 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 each times 10⁻⁹, 10⁻⁸10⁻⁷10⁻⁷10⁻⁶10⁻⁵ 10⁻⁴10⁻³10⁻²10⁻¹ molar per dose. Preferably, Non-Muscle Mysosin II antagonist is present in single dose at a concentration between about 1-100, 2-60, 3-50, 4-40, 5-30, 6-20, 7-15, 8-10, 2-18, 3-16, 4-14, 5-12, 6-10 or 7-8 µM.

In certain embodiments, the therapeutic compositions disclosed herein are administered in a dose about or at least or more than 1, 2, 3, 4, 5, 6, 7, 8, 9, 10, 11, 12, 13, 14, 15, 16, 17, 18, 19, 20, 21, 22, 23 or 24 times per day, week or month over a period of about or at least or more than 1, 2, 3, 4, 5, 6, 7, 8, 9, 10, 11, 12, 13, 14, 15, 16, 17, 18, 19, 20, 21, 22, 23 or 24 days, weeks or months.

In certain embodiments, the NM II antagonist is incubated with the cells of the composition about, at least or more than 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 or 99 minutes, hours or days prior to administration to the patient.

Certain embodiments involve a kit comprising cells and an NM II antagonist. Certain kit embodiments comprise (a) a container that contains at least one NM II antagonist; and (b) a container having stem cells. The NM II may be in solution or in lyophilized form. The kit may optionally include instructions for (i) use of the solution or (ii) reconstitution and/or use of the lyophilized formulation; or (iii) using the contents to treat cardiovascular disease. The kit may further comprise one or more of (iii) a buffer, (iv) a diluent, (v) a filter, (vi) a needle, (v) a syringe, or (vi) an automated medical device. In one embodiment, the container is selected from the group consisting of: a bottle, a vial, a syringe, a test tube, or a multi-use container. In another embodiment, the target peptide composition is lyophilized.

A single dose of the therapeutic compositions described herein can contain at least or about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100 each times 1, 10, 10², 10³, 10¹, 10¹, 10⁶, 10¹, 10¹, 10⁹, 10¹⁰ or more cells per kilogram of patient body weight. In certain embodiments, a dose contains about, at least or more than 1-20, 2-19, 3-18, 4-17, 5-16, 6-15, 7-14, 8-13, 9-12 or 10-11 each times 10⁶ stem cells.

In certain embodiments, the addition of the NM II antagonist in the inventive compositions allow the clinician to provide a patient with a lower dose of stem cells than would be otherwise be needed to achieve the same therapeutic effect achieved with a population of stem cells lacking the NM II antagonist. In certain embodiments, the addition of the NM II antagonist in the inventive compositions allow the clinician to provide a patient with at least or about 5, 10, 15, 20, 25, 30, 35, 40, 45, 50, 55, 60, 65, 70, 75, 80, 90 or more percent less stem cells per dose than would be otherwise be needed to achieve the same therapeutic effect over a define period of time.

Disclosed herein are both methods of and compositions for treating cardiovascular disease; and methods of reducing the complications (e.g., amputation) of treating cardiovascular disease when employing MSCs. Moreover, kits including cells, NM II antagonists, pharmaceutically acceptable carriers, and - optionally - appropriate instructions, are disclosed.

The invention also relates to a method of reducing the rate of amputation in a peripheral artery disease patient treated with a composition comprising MSCs, comprising contacting the MSCs with a non-muscle myosin II antagonist prior to the treatment. Ideally, the non-muscle myosin II antagonist is blebbistatin.

In some embodiments, the methods of treating can generally involve intramuscular injection of a dose of a composition of MSCs treated with an NM II antagonist such as blebbistatin at or near the site of the lesions arising from CVD such as PAD. Such injections generally result in an increase in limb function, blood flow, and ischemic symptoms. Moreover, the use of an NM II antagonist such as blebbistatin in the composition dramatically accelerates MSC-triggered regeneration of damaged tissues. Furthermore, application of blebbistatin with MSCs drastically reduces severe complications associated with PAD and/or the transplantation of MSCs to a patient suffering from PAD.

The methods and kits can also include additional cardiovascular drugs or medical devices such as for example stents (which may or may not be drug eluting). In another embodiment, such stents may be loaded with MSCs which have been contacted with NM II antagonists prior to or during implantation.

### Additional References:

Armstrong, L., Lako, M., Buckley, N., Lappin, T. R. J., Murphy, M. J., Nolta, J. a, Pittenger, M., et al. (2012). Editorial: Our top 10 developments in stem cell biology over the last 30 years. Stem cells (Dayton, Ohio), 30(1), 2-9. doi:10.1002/stem.1007
Ren G, Chen X, Dong F, Li W, Ren X, Zhang Y, Shi Y: Concise review: mesenchymal stem cells and translational medicine: emerging issues. Stem Cells Transl Med. 2012 Jan;1(1):51-8. Review.
Masuzawa K, Jesmin S, Maeda S, Kaji Y, Oshika T, Zaedi S, Shimojo N, Yaji N, Miyauchi T, Goto K. A model of retinal ischemia-reperfusion injury in rats by subconjunctival injection of endothelin-1. Exp Biol Med (Maywood). 2006 Jun;231(6):1085-9.
Straight AF, Cheung A, Limouze J, Chen I, Westwood NJ, Sellers JR, Mitchison TJ. Dissecting temporal and spatial control of cytokinesis with a myosin II Inhibitor. Science. 2003 Mar 14;299(5613):1743-7.
Walker A, Su H, Conti MA, Harb N, Adelstein RS, Sato N. Non-muscle myosin II regulates survival threshold of pluripotent stem cells.Nat Commun. 2010 Sep 7;1:71.
Daigh, Christine; Fuhrken, Peter; Salvagiotto, Giorgia: Method and composition for the differentiation of stem cells. US20100216181A1, US Patent application 2010
Schenk, Judith; van den Bos, Christian; Rosenbaum, Claudia; Nie, Ying: Method for controlling binding of cells to a substrate. 2012 WO2012062819, Patent application
Cai L, Johnstone BH, Cook TG, Liang Z, Traktuev D, Cornetta K, Ingram DA, Rosen ED, March KL..Suppression of hepatocyte growth factor production impairs the ability of adipose-derived stem cells to promote ischemic tissue revascularization. Stem Cells. 2007 Dec;25(12):3234-43. Epub 2007 Sep 27
Goto T. , Fukuyama N., Aku A., Kanabuchi K. ,Kimura K. , Taira H., Tanaka E." Wakana N., Mori H., and Inoue H.. Search for appropriate experimental methods to create stable hind-limb ischemia in mouse. Tokai J Exp Clin Med., Vol. 31, No. 3, pp. 128-132, 2006
OECD principles of Good Laboratory Practice ENV/MC/CHEM (98)17.
Stabile E., Burnett M.S., Watkins C., Kinnaird T, Bachis A., la Sala A., Miller J.M., Shou M., Epstein S.E., Fuchs S. Impaired arteriogenic response to acute hindlimb ischemia in CD4-knockout mice. Circulation. 2003;15;108(2):205-210

### EXAMPLES

### Example 1 - Experimental Set Up

Disclosed herein is the testing of BM MSCs in a mouse model for hind limb peripheral ischemia. Peripheral artery disease (PAD) is a form of peripheral vascular diseases (PVD) in which there are partial or total blockage of blood supply to a limb, usually the leg, leading to impaired blood flow and hypoxia in the tissue. When PAD advances it reaches the stage of critical limb ischemia (CLI) with skin ulcerations, gangrene and unavoidable amputations. One of the most promising innovative treatments for diabetic vascular complications is stem cell-based products that enhance angiogenesis (TRef1-3) Hind limb ischemia animal models have been used to evaluate various therapeutic approaches addressing stem cell transplantation.

Animal handling was according to the National Institute of Health (NIH) and the Association for Assessment and Accreditation of Laboratory Animal Care (AAALAC). Animals were housed in polysufone (PSU) cages (7-8/cage) measuring 42.5 × 265.6 × 18.5 cm, with stainless steel top grill having facilities for pelleted food and drinking water in glass-clear polycarbonate bottle; bedding: steam sterilized clean paddy husk (Harlan, Sani-chip, Cat#: 106S8216) was used and bedding material was changed along with the cage at least twice a week. Animals were fed *ad libitum* a commercial rodent diet (Teklad Certified Global 18% Protein Diet cat #: 106S8216). Animals have free access to autoclaved and acidified drinking water (pH between 2.5 and 3.5) obtained from the municipality supply. Animals were housed under standard laboratory conditions, air conditioned and filtered (HEPA F6/6) with adequate fresh air supply (Minimum 15 air changes/hour). Animals were kept in a climate controlled environment. Temperatures range was 20-24°C and RH range was 30-70% with 12 hours light and 12 hours dark cycle. Species/Strain: Mouse/Athymic Nude; Gender /Number/Age: Male/152/9-10 weeks; Source: Harlan Laboratories, Israel; Body weight: The average body weight was 28.4 g at study initiation (day 1). The minimum and maximum weights of the group were within a range of ±20 % of group mean weight; Acclimation period: 5-10 days; Identification: Three position ear notching and cage cards. No animals was found in a moribund condition or showed severe pain and enduring signs of severe distress. Surgery Day was defined as "DAY 0" in this study. On the day of surgery anesthesia was induced by 1.5 to 3.0% isoflurane, 1.5% N₂O and 0.5% O₂.

Test items: Human Bone Marrow Mesenchymal Stem Cells (Lonza Cologne GmbH). Vehicles: DMEM + 10 % FCS, Optional: 10 µM Blebbistatin (BB).

Under anesthesia, the mouse was placed with ventral side up. A 0.5-1.0 cm incision was made in the skin in the inguinal area. The femoral artery was ligated twice with 6-0 silk thread and transected between the ligatures. The wound was closed with 4-0 silk thread and the mouse was allowed to recover.

On DAY 1, 24 hours post-surgery, each treated animal was injected intramuscular at two sites (the proximal and the distal side of the surgical wound) or intravenous into the tail vein. For intramuscular injection the animals were injected 50 µl at each site, total 100 µl per animal. For intravenous injection the animals were injected by 200 µl per animal. See Table 1 for Groups Allocation.

**Table 1 Groups allocation**

| **Group** | **Number of Animals** | **Treatment (Lot)** | **Treatment Day** | **Cell Amount** | **Route of Administration** |
|---|---|---|---|---|---|
| 1 | 15 | DMEM | (24hrs post-surgery) | 0 | i.m. into 2 sites |
| 2 | 17 | DMEM + 10µM BB | | 0 | |
| 3 | 15 | BM.MSC in DMEM | | 0.25x10⁵ in 0.1mL | |
| 4 | 15 | BM.MSC in DMEM + 10µM BB | | 0.25x10⁵ in 0.1mL | |
| 5 | 15 | BM.MSC pre-treated with BB injected in DMEM | | 0.25x10⁵ in 0.1mL | |
| 6 | 15 | DMEM | | 0 | i.v in tail vein |
| 7 | 15 | DMEM + 10µM BB | | 0 | |
| 8 | 15 | BM.MSC in DMEM | | 0.25x10⁵ in 0.2mL | |
| 9 | 15 | BM.MSC in DMEM + 10µM BB | | 0.25x10⁵ in 0.2mL | |
| 10 | 15 | BM.MSC pre-treated with BB injected in DMEM | | 0.25x10⁵ in 0.2mL | |

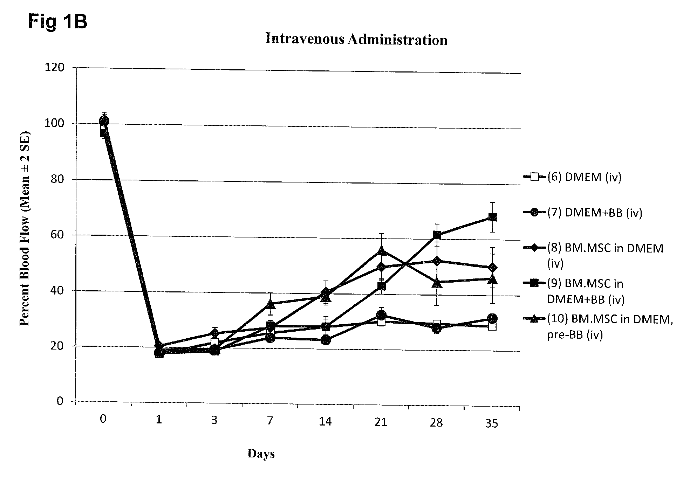

Body weight was measured on study day -1 prior to the surgery and once weekly thereafter. Blood flow measurement: Blood flow in legs from both sides was measured with a non-contact Laser Doppler before surgery and on days: 1, 3, 7, 14, 21, 28 and 35 post operation. Blood flow measurements were expressed as the ratio of the flow in the ischemic limb to that in the normal limb (Goto et al. Tokai J Exp Clin Med., Vol. 31, No. 3, pp. 128-132, 2006). Consistently best regeneration was shown in Group 4, animals treated with MSC+BB (Fig.1). Macroscopic evaluation of ischemic severity: Macroscopic evaluation of the ischemic limb was performed once a week post operation by using morphological grades for necrotic area (Goto et al.): See Table 2.

**Table 2 Morphological grades for necrotic area**

| **Grade** | **Description** |
|---|---|
| 0 | absence of necrosis |
| 1 | necrosis limiting to toes (toes loss), |
| 2 | necrosis extending to a dorsum pedis (foot loss), |
| 3 | necrosis extending to a crus (knee loss) |
| 4 | necrosis extending to a thigh (total hind-limb loss) |

In vivo assessment of limb function and Ischemic damage. Semi-quantitative assessment of impaired use of the ischemic limb was performed once a week post-surgery using the following scale (Stabile et al., Circulation. 2003;15;108(2):205-210). See Table 3.

**Table 3 Assessments of limb function**

| **Grade** | **Description** |
|---|---|
| 0 | flexing the toes to resist gentle traction of the tail |
| 1 | plantar flexion |
| 2 | no dragging but no plantar flexion |
| 3 | dragging of foot |

Limb function is graded as "Not applicable" in case of partial or full limb amputation. In such case blood flow measurements was not included in the statistical analysis.

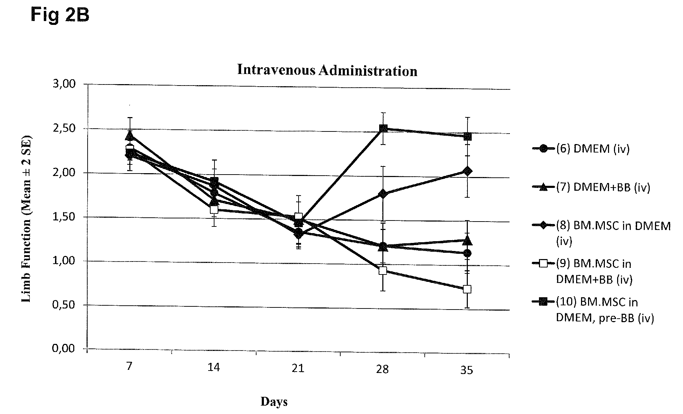

In vivo assessment of limb function. Semi-quantitative assessment of impaired use of the ischemic limb was performed on days 7 up to 35 by using graded functional scales. An improvement in limb function starting from day 14 was found in animals from group 4 treated with cells versus control animal groups 1 and 2 (Fig 2A). That improvement was found in animals groups 3 and 5 on day 35 after ischemia. In animals administered test article intravenously, improvement in limb function starting from day 28 was found in group 9 compare to control groups 6 and 7 (Fig 2B).

### EXAMPLE 2 - Statistical Analysis

The purpose of this study was to test the efficacy of the tested stem cells as a possible treatment to alleviate HLI symptoms in an animal nude mouse model.

Study end points were: Clinical score; Body weight measurements; Blood flow measurements Ranking analysis: Each group was given a ranking from 1 to 10 on each of the first 3 endpoints measured (% Blood Flow, Limb Function and Ischemic Severity Score). Rank=1 is highest and Rank=10 is lowest. When two groups are tied, both receive the same rank "in the middle"; e.g. if two groups were tied in Rank = 4, both received the score 4.5 (twice of which is the sum of ranks 4 and 5). For each endpoint, the median was computed for each group (the median was used to ensure that extreme value will not affect results). On each endpoint, groups were sorted by median and each given its respective rank. The average rank was computed for each group over all endpoints

Statistical testing was done using Mixed Model in SAS®. Main comparisons presented are between the two most effective treatments and all the others. Full statistical testing results are provided herein. P-values provided both in the report and in the appendixes are not adjusted for multiplicity. To determine significance, use P < 0.0056. There are many ways to adjust for multiple testing here based on the interpretation of tests that are meaningful and those that are not. Since this is an exploratory trial, it was decided that adjustment would be considered within each group separately. Thus, for example, Group 4 is compared to 9 other groups so that we determine that a significant result is P < 0.05/9 = 0.0056.

Slope: The slope computed is of the linear trend from surgery to Day 35. This single number for each animal represents its change on an endpoint over time. Distribution of slopes is provided for each group on each of the endpoints (excluding Weight), using Box Plots. These plots provide a convenient method for presenting many groups on a single graph. They should be read as follows: 25% of data points (in our case, slopes) are between the lower hinge and the bottom of the box-percentiles 0 to 25; 50% of data points are located between the bottom and top of the box-percentiles 25 to 75. This is also termed the "inter-quartile range" (IQR). 25% of data points are located between top of the box and upper hinge-percentiles 75 to 100. However, upper and lower fences are defined by a distance of 1.5*IQR from bottom and top of the box. While they are not actually shown in the plot, values outside these fences are considered outliers. So if there are data points above the upper fence, or below the lower fence, they are indicated separately on the boxplot; in other words, extreme points are not included between lower and upper hinges of the boxplot.

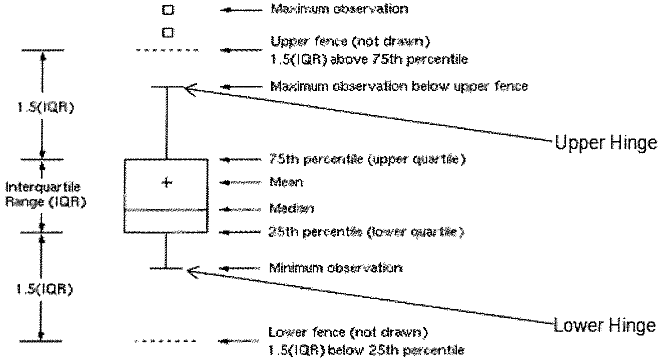

**Table 4 Animal Disposition**

| Treatment Group | Disposition | | | | | | | |
|---|---|---|---|---|---|---|---|---|
| | Excluded¹ | | Imputed² | | Completed³ | | Total | |
| | N | % | N | % | N | % | N | % |
| (1) DMEM (im) | 1 | 6.7 | 1 | 6.7 | 13 | 86.7 | 15 | 100.0 |
| (2) DMEM+BB (im) | 3 | 17.6 | 0 | 0 | 14 | 82.4 | 17 | 100.0 |
| (3) BM.MSC in DMEM (im) | 0 | 0 | 6 | 40.0 | 9 | 60.0 | 15 | 100.0 |
| (4) BM.MSC in DMEM+BB (im) | 1 | 6.7 | 0 | 0 | 14 | 93.3 | 15 | 100.0 |
| (3) BM.MSC in DMEM, pre-BB (im) | 0 | 0 | 1 | 6.7 | 14 | 93.3 | 15 | 100.0 |
| (6) DMEM (iv) | 1 | 6.7 | 0 | 0 | 14 | 93.3 | 15 | 100.0 |
| (7) DMEM+BB (iv) | 1 | 6.7 | 0 | 0 | 14 | 93.3 | 15 | 100.0 |
| (8) BM.MSC in DMEM (iv) | 0 | 0 | 5 | 33.3 | 10 | 66.7 | 15 | 100.0 |
| (9) BM.MSC in DMEM+BB (iv) | 0 | 0 | 1 | 6.7 | 14 | 93.3 | 15 | 100.0 |
| (10) BM.MSC in DMEM, pre-BB (iv) | 2 | 13.3 | 7 | 46.7 | 6 | 40.0 | 15 | 100.0 |
| Total | 9 | 5.9 | 21 | 13.8 | 122 | 80.3 | 152 | 100.0 |

| | | | | | | | | |
|---|---|---|---|---|---|---|---|---|
| 1. Excluded from all further analyses due to either: a) Amputation before or at day 14 following surgery - attributed to model failure, b) Death unrelated to treatment. 2. Imputed due to amputation after day 14, i.e. starting from day 21- attributed to treatment failure. 3. Completed35 days follow-up. | | | | | | | | |

In further analyses the imputation of missing data is done in the following way: Blood Flow (%): Value of the first measurement after the surgery (assumption is that treatment failed so that there is no improvement beyond what was observed immediately after surgery); Ischemic Severity Score: 4="Total Hind-Limb Loss" (the worst score possible); Limb Function: 3="Dragging of foot" (the worst score possible); Weight (g): The animal's lowest weight at any of the points measured up to and including the day of amputation.

While paired comparisons were done, central results are presented in a way in that they are statistically relevant and can be clearly depicted in graphics. Data are consistent across endpoints and are consistent across different analysis. To obtain this picture we eliminated Weight from consideration. Descriptive statistics and testing indicated that Weight was not a meaningful measure of efficacy in the model. Each group was ranked on remaining 3 endpoints on day 35 and assessed Average Rank of groups - a measure combining results from all endpoints.

Evaluating all Parameters Concurrently at Day 35. Procedure: For each parameter (Blood Flow (%), Limb Function and Ischemic Severity Score) the median in the group across all animals was computed. All the groups based on their median from 1 to 10, where 1 is best, were ranked on each of the following parameters: Blood Flow (%)-highest is 1, lowest is 10; Limb Function-highest is 10, lowest is 1 (worst category is 3, best is 0); Ischemic Severity Score-highest is 10, lowest is 1 (worst category is 4, best is 0); Finally, for each group we computed the average rank over the three parameters.

**Table 5 Ranking of Treatment Groups on Day=35, Imputed Data**

| **Group** | Blood Flow (%) | Limb Function | Ischemic Severity Score | Average Rank | Factor* | Factor 2** |
|---|---|---|---|---|---|---|
| (4) Treatment: BM.MSC in DMEM+BB (im) | 1 | 1 | 1.5 | 1.2 | | |
| (5) Treatment: BM.MSC in DMEM, pre-BB (im) | 3 | 5 | 1.5 | 3.2 | 2.0 | 2.0 |
| (9) Treatment: BM.MSC in DMEM+BB (iv) | 2 | 5 | 6 | 4.3 | 3.2 | 1.2 |
| (3) Positive Control: BM.MSC in DMEM (im) | 4 | 5 | 6 | 5.0 | 3.8 | 0.7 |
| (2) Negative Control: DMEM+BB (im) | 6.5 | 5 | 6 | 5.8 | 4.7 | 0.8 |
| (7) Negative Control: DMEM+BB (iv) | 65 | 5 | 6 | 5.8 | 4.7 | 0.0 |
| (6) Negative Control: DMEM (iv) | 8 | 5 | 6 | 6.3 | 5.2 | 0.5 |
| (1) Negative Control: DMEM (im) | 9 | 5 | 6 | 6.7 | 5.5 | 0.3 |
| (8) Positive Control: BM.MSC in DMEM (iv) | 5 | 9.5 | 6 | 6.8 | 5.7 | 0.2 |
| (10) Treatment BM.MSC in DMEM, pre-BB (iv) | 10 | 9.5 | 10 | 9.8 | 8.7 | 3.0 |

| | | | | | | |
|---|---|---|---|---|---|---|
| Median per treatment group was used * Factor by which average rank differs from the top-ranked group (4) ** Factor 2 - Difference between ranks of adjacent-ranked groups | | | | | | |

From the table it can be seen that there are four general magnitudes of rank:
Group 4 (BM.MSC in DMEM+BB (im)) has the highest rank and is far ahead of the 2nd most effective group (5): Average Rank = 1.2, difference from next group is 2.
Group 5 is far behind Group 4 (as indicated in the bullet above) but also ahead of the groups behind it. Specifically, it is 1.2 ranks ahead of the 3rd most effective group (9).

The next seven groups (9, 3, 2, 7, 6, 1 and 8) are in the 3rd category with a difference between best and worst - 2.5 (6.8-4.3), which is equivalent smaller than the distance between 1 st and 3rd places (3.1).

Group 10 (BM.MSC in DMEM, pre-BB (iv)) is far behind the "block of seven groups" in the previous bullet - 3 ranks. Note that Group 10 turned out especially ineffective due to a large number of amputations and so, ineffective values imputed for Day 35.

In Table 6 the results of the Significance Testing are depicted. The two best groups are compared with all others on all endpoints.

**Table 6 Pairwise Comparisons Groups 4 and 5 versus all others, Imputed Data**

| | Blood Flo (%) | | Limb Function | | Ischemic Severity Score | |
|---|---|---|---|---|---|---|
| Treatment Group | (4) | (5) | (4) | (5) | (4) | (5) |
| (1) Negative Control: DMEM (im) | <.0001 | <.0001 | 0.0014 | 0.9614 | 0.0208 | 0.2723 |
| (2) Negative Control: DMEM+BB (im) | <.0001 | <.0001 | <.0001 | 0.2760 | 0.0509 | 0.4688 |
| (3) Positive Control: BM.MSC in DMEM (im) | <.0001 | 0.0034 | <.0001 | 0.4911 | <.0001 | 0.0008 |
| (4) Treatment: BM.MSC in DMEM+BB (im) | --- | 0.0001 | --- | 0.0010 | --- | 0.2036 |
| (5) Teatment: BM.MSC in DMEM, pre-BB (im) | 0.0001 | --- | 0.0010 | --- | 0.2036 | --- |
| (6) Negaive Control: DMEM (iv) | <.0001 | <.0001 | <.0001 | 0.0709 | 0.0025 | 0.0660 |
| (7) Negative Control: DMEM+BB (iv) | <.0001 | <.0001 | <.0001 | 0.0208 | <.0001 | 0.0050 |
| (8) Positive Control: BM.MSC in DMEM (iv) | 0.0002 | 0.9142 | <.0001 | <.0001 | <.0001 | <.0001 |
| (9) Treatment: BM.MSC in DMEM+BB (iv) | 0.0002 | 0.9442 | <.0001 | 0.4314 | 0.0003 | 0.0137 |
| (10) Treatment: BM.MSC in DMEM, pre-BB (iv) | <.0001 | 0.7473 | <.0001 | <.0001 | <.0001 | <.0001 |

| | | | | | | |
|---|---|---|---|---|---|---|
| **Note: P-values less than 0.05 are underlined** | | | | | | |

Impaired angiogenesis is one of the features of ischemic diseases. However, clinical trials to alleviate ischemia have been disappointing, indicating the need for new molecules and therapeutic targets to treat ischemic diseases. Stem cell therapy is a promising approach in cardiovascular medicine. In order to assess the therapeutic activity of stem cells in ischemic tissue, the mouse hind limb ischemia model is used.

It is disclosed herein that that MSCs showed regenerative potential in an animal mouse model, whereas the substance Blebbistatin alone had no beneficial effect. This validated the model. In these experiments, a single IV or local administration to the ischemic limb of the tested cells surprisingly and unexpected, restored blood perfusion up to 65-84% of its normal values. Blood flow restoration was compatible with other results showing improvement in limb function and decreased and delayed ischemic severity in the mouse hind-limb ischemia model. This effect was achieved in the groups treated with tested cells locally (groups 4 and 5) or intravenously (group 9).

Blebbistatin dramatically accelerated MSC-triggered regeneration of damaged tissues. Application of Blebbistatin with MSCs drastically reduces severe complications in healing as clearly demonstrated by Group 4 (BM.MSC in DMEM +BB (im)) which is more effective than all groups on % Blood Flow and Limb Function, including group 5 BM.MSC in DMEM, pre-BB (im). In addition, Blebbistatin drastically improves the regenerative potential of MSCs by reducing severe complications in healing.

Group 4 (BM.MSC in DMEM +BB (im) is more effective than all groups on % Blood Flow and Limb Function, including group 5 BM.MSC in DMEM, pre-BB (im).

The results presented herein demonstrate that application of BM.MSC in the presence of Blebbistatin provides greatest efficacy when applied intramuscular. This combined application showed fastest recovery from the lesion resulting in the most effective treatment. The relatively small variation in the best group indicates consistency of efficacy across animals. Unless defined otherwise, all technical and scientific terms and any acronyms used herein have the same meanings as commonly understood by one of ordinary skill in the art in the field of this invention. Although any compositions, methods, kits, and means for communicating information similar or equivalent to those described herein can be used to practice this invention, the preferred compositions, methods, kits, and means for communicating information are described herein.

## Claims

1. A composition comprising a population of stem cells and blebbistatin for use in treating a disease amenable to treatment with stem cell therapy.

2. Composition for use according to claim 1, wherein the disease is selected from the group of, a disease that is a result of diabetes, a disease that is a result of atherosclerosis, a vascular disease, a peripheral artery disease (PAD).

3. Composition for use according to claim 1 or 2, wherein the disease is associated with an ankle brachial pressure index of about or less than 0.9, or an ankle brachial pressure index of about or less than 0.7.

4. Composition for use according to claims 1 to 3, wherein the disease has resulted in critical limb ischemia and/or the disease has resulted in skin ulcerations or gangrene.

5. Composition for use according to claims 1 to 4, wherein a portion of the population of cells comprises pluripotent cells and/or, wherein the pluripotent cells are induced pluripotent stem cells, and/or, wherein a portion of the population of cells comprises multipotent cells and/or, wherein the multipotent cells are multipotent stromal cells or mesenchymal stem cells and/or, wherein the multipotent cells are derived from induced pluripotent stem cells.

6. Composition for use according to any of claims 1 to 5, wherein the stem cells are MSCs.

7. A kit for treating a disease amenable to treatment with stem cell therapy comprising:
a. blebbistatin:
b. a pharmaceutically acceptable carrier;
c. a population of stem cells; and
d. optionally instructions for administering items (a)-(c) to a patient diagnosed with cardiovascular disease.

8. The kit of claim 7, wherein the cells are multipotent cells.

9. The kit of claim 7, wherein the cells are MSCs.

## Patentansprüche

1. Zusammensetzung umfassend eine Population von Stammzellen und Blebbistatin zur Verwendung bei der Behandlung einer Krankheit, die auf eine Behandlung mit Stammzellentherapie anspricht.

2. Zusammensetzung zur Verwendung nach Anspruch 1, wobei die Krankheit ausgewählt ist aus der Gruppe, bestehend aus einer Krankheit, die das Ergebnis eines Diabetes ist, einer Krankheit, die das Ergebnis von Atheriosklerose ist, einer Gefäßkrankheit, einer peripheren Arterienkrankheit (peripheral artery disease, PAD).

3. Zusammensetzung zur Verwendung nach Anspruch 1 oder 2, wobei die Krankheit mit einem Knöchel-Arm-Druckindex von etwa 0,9, oder weniger, oder einem Knöchel-Arm-Druckindex von etwa 0,7, oder weniger, zusammenhängt.

4. Zusammensetzung zur Verwendung nach den Ansprüchen 1 bis 3, wobei die Krankheit zu einer kritischen Gliedmaßenischämie führte und/oder die Krankheit zu Hautgeschwüren oder Wundbrand führte.

5. Zusammensetzung zur Verwendung nach den Ansprüchen 1 bis 4, wobei ein Teil der Population der Zellen pluripotente Zellen umfasst, und/oder wobei die pluripotenten Zellen induzierte pluripotente Stammzellen sind, und/oder wobei ein Teil der Population der Zellen multipotente Zellen umfasst, und/oder wobei die multipotenten Zellen multipotente Stromazellen oder mesenchymale Stammzellen sind, und/oder wobei die multipotenten Zellen von induzierten pluripotenten Stammzellen abstammen.

6. Zusammensetzung zur Verwendung nach einem der Ansprüche 1 bis 5, wobei die Stammzellen MSCs sind.

7. Kit zur Behandlung einer Krankheit, die auf die Behandlung mit Stammzellentherapie anspricht, umfassend:
a) Blebbistatin;
b) einen pharmazeutisch annehmbaren Träger;
c) eine Population von Stammzellen; und
d) optional Anweisungen zur Verabreichung der Objekte (a) bis (c) an einen Patienten, bei dem eine Herz-Kreislauferkrankung diagnostiziert wurde.

8. Kit nach Anspruch 7, wobei die Stammzellen multipotente Zellen sind.

9. Kit nach Anspruch 7, wobei die Zellen MSCs sind.

## Revendications

1. Composition comprenant une population de cellules souches et de la blebbistatine pour son utilisation dans le traitement d'une maladie pouvant être traitée avec une thérapie par les cellules souches.

2. Composition pour son utilisation selon la revendication 1, dans laquelle la maladie est sélectionnée dans le groupe d'une maladie qui est un résultat du diabète, d'une maladie qui est un résultat de l'athérosclérose, d'une maladie vasculaire, d'une artériopathie périphérique (PAD).

3. Composition pour son utilisation selon la revendication 1 ou 2, dans laquelle la maladie est associée à un indice de pression systolique cheville-bras d'environ 0,9 ou inférieur à 0,9, ou à un indice de pression systolique cheville-bras d'environ 0,7 ou inférieur à 0,7.

4. Composition pour son utilisation selon les revendications 1 à 3, dans laquelle la maladie a résulté en une ischémie des membres critique et/ou la maladie a résulté en une ulcération de la peau ou la gangrène.

5. Composition pour son utilisation selon les revendications 1 à 4, dans laquelle une partie de la population de cellules comprend des cellules pluripotentes et/ou, dans laquelle les cellules pluripotentes sont des cellules souches pluripotentes induites, et/ou, dans laquelle une partie de la population de cellules comprend des cellules multipotentes et/ou dans laquelle les cellules multipotentes sont des cellules stromales multipotentes ou des cellules souches mésenchymateuses et/ou, dans laquelle les cellules multipotentes sont dérivées de cellules souches pluripotentes induites.

6. Composition pour son utilisation selon l'une quelconque des revendications 1 à 5, dans laquelle les cellules souches sont des MSC.

7. Kit de traitement d'une maladie pouvant être traitée par une thérapie par les cellules souches comprenant :
a. de la blebbistatine ;
b. un vecteur pharmaceutiquement acceptable ;
c. une population de cellules souches ; et
d. facultativement des instructions pour l'administration des points (a) à (c) à un patient à qui une maladie cardiovasculaire a été diagnostiquée.

8. Kit selon la revendication 7, dans lequel les cellules sont des cellules multipotentes.

9. Kit selon la revendication 7, dans lequel les cellules sont des MSC.
